# EUROPEAN PATENT APPLICATION

(11) **EP 4 102 515 A1**
(43) Date of publication of application: **14.12.2022**
(21) Application number: 21179031.6
(22) Date of filing: 11.06.2021
(51) Int. Cl.: G16H 40/20

(54) **METHOD AND DEVICES FOR DIRECTING A USER TO A SUBJECT IN NEED OF CARE**

(71) Applicant: AssistMe GmbH, 10555 Berlin (DE)
(72) Inventor: Carucci Lemke, Diego, 10245 Berlin (DE)
(74) Representative: Gulde & Partner

(57) **Abstract**

The invention relates to a method for directing a user (2) to a subject (1) in need of care, the method being of a controller of an electronic device. As a step (S1) the method comprises an outputting of information (i1) on the subject (1) in need of care to the user (2). In another step (S2) information on a current location of the user (2) are obtained. A further step (S3) is determining a route from the current location to a location of the subject (2) and in another step (S4) navigation information (n1, n2) is output based on the determined route to the user (2). Another aspect of the invention is a system comprising a controller configured to perform the method, at mobile device (20) and localisation means (30). The invention further relates to a controller that is configured to perform the inventive method and to a mobile device (20) comprising such a controller.

## Description

The invention relates to a method for directing a user to a subject in need of care by a controller of an electronic device. Further it relates to a system comprising a controller configured to perform the method, a mobile device and localisation means. The invention further relates to such a controller itself, to a mobile device comprising or communicating with such controller and to a software program for operating such controller.

One of the biggest challenges that modern societies have to face in coming years is the aging of their populations. The number of caregivers and elderly at nursing homes will become less balanced, therefore less caregivers will attend more residents, which raises several difficulties.

Already today caregivers do check on residents according to a tight time schedule, not only making it an exhausting workload but also rendering it difficult to figure out, which urgent need to attend first. Also important to note is that caregivers have assigned a certain number of residents they have to monitor on each shift, which renders efficient scheduling even more important.

Another current pain point at care homes are night shifts, wherein one of the main challenges is the wide area of the care home, and the reduced numbers of caregivers during these shifts. Therefore the physical strain on caregivers becomes a problem.

Further, due to the lack of caregivers, it often occurs that caregivers have to fill in for others or are hired on a temporary basis from external sources. In such cases, it is important to ensure rapid familiarization and thus smooth integration into the nursing home's workflow.

It is thus an object of the present disclosure to overcome or reduce at least some of the drawbacks of the prior art and to provide a method and system for directing a user to a subject in need of care to improve the workflow in nursing facilities and nursing homes in particular.

The given task is solved by the subject-matter of the independent claims. Advantageous embodiments of the invention can further be gained from the dependent claims.

A first aspect of the disclosure refers to a method for directing a user to a subject in need of care by a controller of an electronic device. A subject, in the sense of the present disclosure, is a human being that regularly requires assistance and/or care, such as a patient of a hospital or a resident of a nursing or care home. A subject in need of care, in the sense of the present disclosure, is a subject with a care need, wherein a care need is a condition of a human being that momentarily requires assistance and/or care. Exemplarily, a care need is an acute condition of a human being that can be improved by an action that cannot be performed by the human being himself or herself, such as, for example, a stool or urine occurrence in an incontinence material that can be improved by replacing the material. Preferably, the subject in need of care is a subject in an acute condition that requires momentary assistance and/or care from a user, i.e., within a certain, e.g., predefined, time.

A user, in the sense of the present disclosure, is a human being who is entrusted with the care of at least one other human being. Preferably, a user is trained to treat a care need. For example, a user is a nurse, a nurse practitioner, a nurse for the elderly, a physician, a doctor, and/or other caregiver. Alternatively preferred, a user is a robot configured to treat a care need. An electronic device, in the sense of the present disclosure, is preferably a portable device, such as a smartphone, data glasses, a smartwatch, virtual reality, VR, or augmented reality, AR, glasses, or a laptop. Further preferably, an electronic device is a stationary device such as a computer or a server of a network.

The method according to the present disclosure comprises a step of outputting information on the subject in need of care to the user. The information preferably comprises an indication on at least one of a care need and a location of the subject in need of care. A location, in the sense of the present disclosure, is preferably a geographical position or a zone, like, for example, a room, a corridor, a floor and/or a station. The information is preferably determined based on sensor information indicative of the care need and a location of the subject in need of care. Further preferably, the information additionally includes personal information about the subject in need of care, such as his or her name.

Further preferred, the information also includes a location and/or personal information of at least one subject determined to be not in a momentary need of care. Preferably, the information about the subject in need of care is emphasized over that about the at least one subject not determined to be in need of care in case that the information includes both. Especially preferred, the determination of the information on the subject in need of care is repeated periodically and thus the information is regularly adapted to the current situation.

A further step of the method according to the present disclosure comprises an obtaining of information on a current location of the user. In other words, information is obtained from which a current location of the user, preferably a geographical position or zone where the user is currently located, can be determined.

In a further step of the method, a route from the current location to a location of the subject is determined. The location of the subject is preferably determined from the information on the subject. Preferably, the route is determined by means of the Dijkstra shortest path algorithm. Further preferably, the route is determined by means of another shortest path algorithm, such as the Bellman-Ford Algorithm, the Floyd-Warshall Algorithm and/or the Johnson's Algorithm. Preferably, map data and/or data on waypoints are used for determining the route.

The method according to the present disclosure further comprises the step of outputting navigation information based on the determined route to the user. The navigation information is preferably comprising instructions that guide the user to follow the route. The navigation information is preferably output to be displayed visually. Further preferably, the navigation information is output in such a way that they can be audibly presented. Preferably, the navigation information is output by using augmented reality, AR. The navigation information preferably comprises text and/or symbols, such as arrows. Further preferably, the navigation information additionally comprises the length of the route.

The method according to the present disclosure preferably comprises a periodical updating of the current location of the user and repeating the steps of determining and outputting until the current location of the user matches the location of the subject in need of care.

By means of the method according to the disclosure, care needs of one or more subjects can advantageously be recognized automatically and transmitted to one or more users. The method further advantageously ensures that the user is guided to the subject in need of care in the fastest way possible. The method is particularly advantageous for caregivers who are being trained and are thus able to find their way quickly. For caregivers in training as well as for experienced caregivers, detours can be avoided and more time can be spent on the care of the subjects in need of care.

In a preferred embodiment of the present disclosure, the method further comprises the step of outputting information on a plurality of subjects in need of care. Preferably, the information comprises an indication on at least one of the care needs and the location of each of the plurality of subjects. The information is preferably ordered based on care needs and/or locations of the subjects. The information on the plurality of subjects is preferably ordered in such a way that the first subject in the order is having a shortest distance to the user and/or a highest care need. The distances between each of the plurality of subjects and the user are preferably determined based on the current location and locations of the subjects. The highest care need is preferably the care need that requires the fastest intervention from the user. Further preferably, the information is ordered additionally based on a responsibility of the user. In other words, the user is preferably responsible for the first subject in the order, for example, is the responsible nurse for him or her. Additionally preferred, the information is ordered further based on a timer indicating a time since the subjects were lastly visited by a user. Preferably the first subject in the order is further having a greatest time since he or she was lastly visited by a user. Preferably, the information is output in such a format that it can be displayed in a way that indicates which subject is the first subject in the order. In other words, the information about the first subject in the order is emphasized over that about the other subjects.

The preferred embodiment further comprises the step of receiving a user input for selecting one of the plurality of subjects. Preferably the first subject in the order is selected based on the received user input. Further preferred, a specific subject of the plurality of subjects is selected based on the received user input. The route is preferably determined from the current location to the location of the selected subject.

The embodiment of the method advantageously leads to an optimization of the care process, since the information is output in such a way that it conveys the subject with the most urgent care need and/or the nearest location to the user so that the user can choose to be quickly guided to this subject. In this way, for example, it is possible to bring to a caregiver's attention an urgent need that is closest to his or her location, avoiding other caregivers to have to move from farther locations inside a care home and the time until an urgent care need is treated is advantageously shortened.

A further preferred embodiment of the method comprises the step of calculating routes from the current location to the location of each of a plurality of subjects. Preferably the shortest routes from the current location to the location of each of the plurality of subjects are calculated using a shortest path algorithm. Further preferred an optimized route along the locations of at least some of the plurality of subjects is determined. In other words, the optimized route is determined in such a way that someone following the optimized route reaches all of the locations of the at least some of the plurality of subjects in order with the most efficient way possible. Preferably the optimized route is determined based on the calculated routes from the current location to the location of each of the plurality of subjects. Further preferred the optimized route is determined further based on routes between the locations of the plurality of subjects. The optimized route is preferably determined along the locations of each of the plurality of subjects.

In the preferred embodiment, information on the at least some of the plurality of subjects in need of care is preferably output ordered based on the optimized route. Preferably, the order of the information output is the same order in which the locations of the subjects are reached when following the optimized route. Additionally or alternatively preferred, the navigation information is output based on the optimized route. The navigation information is preferably comprising instructions that guide the user to follow the optimized route.

With this preferred embodiment of the method, the user is advantageously not only supported in selecting the next subject in need of care based on a prioritization, but even receives a suggestion for an optimized order to care for all subjects one after the other. Thus, the care process is further optimized and the time until a care need is treated is advantageously reduced while at the same time the time for a treatment of a care need is increased. Advantageously, the user can decide which of the subjects in need of care he or she would like to be directed to next. For example, he or she can select a highest order subject of the plurality of subjects to follow the optimized route. Alternatively, the user can be automatically guided along the optimized route without having to select each subject individually.

In a further preferred embodiment, the optimized route is determined to be optimized with respect to its length and/or a preference for urgent care needs. In other words, the optimized route is determined to include the shortest path on which all locations of the at least some of the plurality of subjects are located and/or locations of subjects with a more urgent care need are located before those of other subjects on the optimized route. The oftentimes difficult decision to which subject in need of care the caregiver should go next is advantageously taken away from him or her by this embodiment. Thanks to the method according to the preferred embodiment, the caregiver can be sure that he or she is guided on the fastest way possible to all subjects in need of care one after the other and is thereby guided first to the subjects who need the help most urgently.

According to an additionally preferred embodiment, the optimized route comprises a supply point for receiving material and/or depositing waste. At the supply point, material is preferably received that is needed to treat a care need, such as new incontinence materials, fresh clothing, fresh bedding, food, or medical products. In addition or alternatively, waste is preferably deposited at the supply point that has been generated during the treatment of a care need, such as used incontinence materials, leftover food, used medical products or packaging thereof, or cleaning waste. Preferably, reusable material can also be dropped off at the supply point for cleaning, such as used dishes or laundry.

An especially preferred embodiment of the method according to the present disclosure envisages that the navigation information is output using augmented reality, AR. The navigation information is preferably output by displaying a camera image of a mobile device by output means thereof overlaid with the navigation information. The mobile device is preferably an AR-enabled device associated with the user and comprising a camera, such as a smartphone, data glasses, or AR or VR glasses. The navigation information is preferably displayed in such a way that it appears to the user to be in his or her real environment. For example, the navigation information is displayed in such a way that it appears to the user as if navigation symbols, in particular arrows, are located in his or her real environment and show him or her the way along the route. Through the preferred embodiment, the user is guided very intuitively to follow the route and thus quickly reach the respective subject in need of care, which advantageously leads to a further improvement of the care process.

Another aspect of the present disclosure relates to a system for directing a user to a subject in need of care. The system preferably comprises a controller configured to perform a method as described above. The controller is preferably established in at least one electronic device. Further preferred the controller is established in a plurality of electronic devices wherein each of the electronic devices is configured to perform a part of the controller's functions. Further preferably, the controller or a part of the controller is established in a cloud server, wherein a cloud server is a server of a network.

The system according to the disclosure further comprises a mobile device associated with the user. Preferably, the system comprises a mobile device worn or hold by the user. The mobile device is preferably a handheld device such as, for example, a smartphone or a tablet, further preferred an augmented reality, AR, device such as, for example, AR glasses, google glasses or an AR-enabled smartphone or tablet, or further preferred a wearable device such as, for example, a pager or a smartwatch.

The mobile device comprises output means configured to display the information on the subject in need of care. The output means are preferably further configured to display information on a plurality of subjects in need of care, wherein the information is displayed in such a way that an order of the subjects is clearly recognizable, for example by means of a colour marking or an arrangement of the information. Further preferred, the output means are configured to display the navigation information to the user. Preferably, the output means are configured to display the navigation information by using augmented reality, AR. The output means are preferably configured to display the navigation information as text and/or symbols, such as arrows. Further preferred, the mobile device comprises input means configured to detect a user input for selecting one of the plurality of subjects.

The mobile device preferably further comprises the controller or is configured to communicate with the controller. The controller is preferably configured to output the information on the subject in need of care and the navigation information to the user by way of the output means of the mobile device. Preferably a part of the controller is established in the mobile device.

Preferably the system further comprises localisation means configured to perform a localisation of the mobile device. The localisation means preferably comprise a plurality of transmitters that are configured to send signals to and/or receive signals from the mobile device to perform the localisation. The signals are preferably wired or wireless communication signals, particularly preferred Bluetooth low energy, BLE, ultra-wideband, UWB or WiFi signals. The plurality of transmitters is preferably evenly distributed at a place of use of the system according to the disclosure, such as, for example, a nursing home. Estimated transmitter locations are preferably arranged in a predetermined geometric pattern based on a number of transmitters in the place of use, wherein the number of transmitters is based on the size of the place of use.

The controller is preferably configured to determine a location of the mobile device based on the localisation, wherein the mobile device is preferably worn or held by the user and thus the location of the mobile device corresponds to the current location of the user. Further preferred, the mobile device is configured to determine its location and the current location based on the localisation. The location of the mobile device is preferably determined based on the strength and/or propagation time of the signals.

The system preferably further comprises at least one wearable sensor associated with at least one subject. Preferably, the system comprises one wearable sensor worn by one subject. Particularly preferably, the system comprises a plurality of wearable sensors worn by a plurality of subjects, each subject of the plurality of subjects wearing one wearable sensor from the plurality of sensors. Preferably, a wearable sensor is attached to an incontinence material worn by a subject. The at least one wearable sensor is preferably configured to detect a location of the at least one subject and sensor information indicating a care need of the at least one subject.

According to a preferred embodiment of the system according to the present disclosure the localisation means comprise a plurality of beacons configured to send beacon signals to the mobile device. In other words, the transmitters are formed as beacons. The beacon signals are preferably wired or wireless communication signals, particularly preferred BLE signals. A beacon signal preferably contains identification data and/or location data of the sending beacon. In this preferred embodiment, the mobile device is configured to receive the beacon signals and to determine location information of the mobile device based on the beacon signals. As the mobile device is preferably worn or held by the user, the location information corresponds to the current location of the user.

The mobile device is preferably configured to determine a location of a beacon based on a received beacon signal that was send by the beacon. Preferably the mobile device is configured to determine the location of the beacon based on the location data and/or the identification data in combination with a location plan of the plurality of beacons. The mobile device is preferably further configured to determine a distance to a beacon based on a strength of a received beacon signal that was send by the beacon. Particularly preferred, the mobile device is further configured to determine the current location based on a determined location of and distance to at least one of the plurality of beacons. Further preferred the mobile device is further configured to determine the current location based on a determined location of and distance to at least three beacons using trilateration. Preferably, the mobile device is configured to determine the current location further based on a model of the place of use of the system containing radio frequency propagation information. In this way, inaccuracies caused by weakening of the beacon signals, e.g. by walls, can advantageously be detected and compensated for.

In a further preferred embodiment of the system, the controller is configured to determine the route based on the Dijkstra shortest path algorithm, wherein each of the plurality of beacons is considered as one node respectively. In other words, the route is determined as the shortest path from beacon to beacon in the network of beacons using the Dijkstra shortest path algorithm. Further preferably, the route is determined by means of another shortest path algorithm, such as the Bellman-Ford Algorithm, the Floyd-Warshall Algorithm and/or the Johnson's Algorithm. Preferably, the route is determined as starting from the current position, continuing to the nearest beacon, then from beacon to beacon, and finally to the location of the subject in need of care.

In a further preferred embodiment, the navigation information indicates a path from the current location to a next beacon along the route. In other words, the navigation information is preferably output in such a way that it guides the user to the next beacon on the route. If the current location matches the location of the last beacon on the route, the navigation information preferably indicates a path from the current location to the location of the subject in need of care.

According to a further preferred embodiment, the controller is configured to update the current location based on determining a proximity of the mobile device and one of the plurality of beacons based on the beacon signals. Preferably, the current location is updated whenever the proximity indicates that the mobile device is in the near distance of one of the plurality of beacons, in particular less than 10 m, preferably less than 5 m, particularly preferably less than 3 m away from the one beacon. Preferably, the controller executes the method steps of determining a route from the current location to a location of the subject and outputting navigation information based on the determined route to the user each time the current location is updated. In this way, the user is advantageously guided along the route from beacon to beacon and a new navigation information is displayed each time he or she reaches a new beacon.

In another preferred embodiment of the system, the mobile device is configured to determine a facing direction and/or a pitch of the mobile device. In this preferred embodiment, the navigation information is output in particular using augmented reality by displaying a camera image of the mobile device by the output means thereof overlaid with the navigation information. Preferably, the mobile device outputs a warning in case the facing direction deviates more than a predetermined threshold from a direction of the navigation information. Additionally or alternatively preferred, the mobile device outputs a warning in case the pitch indicates that the orientation of the mobile device deviates more than a predetermined threshold from an upright position. Preferably, the warning is output instead of the navigation information. In other words, in this preferred embodiment, a warning is issued if the mobile device is in a spatial position in which it cannot be ensured that the camera image obtained in this position depicts a part of the user's surroundings over which the navigation information can be layered to achieve an AR navigation information output. This preferred embodiment advantageously ensures that the AR navigation information is perceived correctly to prevent misunderstandings and to safely guide the user to follow the route to reach the subject in need of care.

Preferred embodiments of the system according to the present disclosure correspond to preferred embodiments of the method according to the disclosure as described above.

Another aspect of the disclosure relates to a controller, preferably a controller of an electronic device, for directing a user to a subject in need of care. The controller is preferably configured to perform the method according to the disclosure as described above.

A further aspect of the disclosure relates to a mobile device for the system according to the disclosure as described above. The mobile device is preferably configured to be hold or worn by the user. The mobile device is preferably a handheld device such as, for example, a smartphone or a tablet, further preferred an augmented reality, AR, device such as, for example, AR glasses, google glasses or an AR-enabled smartphone or tablet, or further preferred a wearable device such as, for example, a pager or a smartwatch. Preferably, the mobile device comprises a controller configured to perform the method according to the invention as described above. Alternatively preferred, the mobile device comprises communication means configured to communicate with a controller configured to perform the method according to the disclosure as described above. The mobile device further comprises output means configured to display the information on the subject in need of care and the navigation information to the user. The controller is preferably further designed to provide the information on the subject in need of care comprising care needs to the user by way of the output means. Preferably, the controller provides information on a plurality of subjects in need of care in such a way that a prioritization of the subjects is clearly recognizable, for example by means of a colour marking or an arrangement of the information.

Preferred embodiments of the mobile device according to the present disclosure correspond to preferred embodiments of the method and system according to the disclosure as described above.

Another aspect of the invention relates to a computer program comprising instructions which, when the program is executed by a computer, such as a controller of an electronic device, cause the computer to execute the method according to the invention as described above.

Such computer programs are typically executed by utilizing the computing resources in a computing device. Applications are stored on a non-transitory medium. An example of a non-transitory medium is a non-volatile memory, for example a flash memory while an example of a volatile memory is RAM. The computer instructions are executed by a processor. These memories are exemplary recording media for storing computer programs comprising computer-executable instructions performing all the steps of the computer-implemented method according the technical concept presented herein.

Another aspect of the invention relates to a computer-readable storage medium comprising instructions which, when executed by a computer, such as a controller of an electronic device, cause the computer to execute the method according to the invention as described above.

Also, a person of skill in the art should recognize that the functionality of various computing devices may be combined or integrated into a single computing device, or the functionality of a particular computing device may be distributed across one or more other computing devices without departing from the scope of the embodiments of the present invention.

The different embodiments of the disclosure described herein may be advantageously combined unless the contrary is indicated herein.

Reference is now made to the following figures, in order to describe preferred embodiments of the invention in more detail.
- Figure 1: illustrates a method for directing a user to a subject in need of care by a controller of an electronic device according to a preferred embodiment of the disclosure;
- Figure 2: illustrates a system for directing a user to a subject in need of care according to a preferred embodiment of the disclosure;
- Figures 3a/3b: illustrate an outputting of information on a subject according to a method in a preferred embodiment of the disclosure;
- Figures 4a/4b: illustrate a user input for selecting one of a plurality of subjects according to a method in a preferred embodiment of the disclosure;
- Figure 5: illustrates an exemplary route of a user according to a preferred embodiment of the disclosure;
- Figures 6a/6b: illustrate an outputting of navigation information according to a method in a preferred embodiment of the disclosure; and
- Figures 7a/7b: illustrate a determination of a pitch by a mobile device according to a preferred embodiment of the disclosure.

Some portions of the detailed description which follows are presented in terms of data processing procedures, steps or other symbolic representations of operations on data bits that can be performed on computer memory. Therefore, a computer executes such logical steps thus requiring physical manipulations of physical quantities.

Usually these quantities take the form of electrical or magnetic signals capable of being stored, transferred, combined, compared, and otherwise manipulated in a computer system. For reasons of common usage, these signals are referred to as bits, packets, messages, values, elements, symbols, characters, terms, numbers, or the like.

Additionally, all of these and similar terms are to be associated with the appropriate physical quantities and are merely convenient labels applied to these quantities. Terms such as "processing" or "creating" or "transferring" or "executing" or "determining" or "detecting" or "obtaining" or "selecting" or "calculating" or "generating" or the like, refer to the action and processes of a computer system that manipulates and transforms data represented as physical (electronic) quantities within the computer's registers and memories into other data similarly represented as physical quantities within the memories or registers or other such information storage.

As utilized herein, the term "example" means serving as a non-limiting example, instance, or illustration. As utilized herein, the terms "for example" and "e.g." introduce a list of one or more non-limiting examples, instances, or illustrations.

As used herein, the term "and/or" includes any and all combinations of one or more of the associated listed items. Further, the use of "may" when describing embodiments of the present disclosure refers to "one or more embodiments of the present disclosure." Further, in the following description of embodiments, the terms of a singular form may include plural forms unless the presented context clearly indicates otherwise.

It will be understood that although the terms "first" and "second" are used to describe various elements, these elements should not be limited by these terms. These terms are only used to distinguish one element from another element. For example, a first element may be named a second element and, similarly, a second element may be named a first element, without departing from the scope of the present invention. As used herein, the term "and/or" includes any and all combinations of one or more of the associated listed items and expressions such as "at least one of" when preceding a list of elements, modify the entire list of elements.

Reference will now be made in detail to embodiments which are illustrated in the drawings. Effects and features of the exemplary embodiments will be described with reference to the accompanying drawings. Therein, like reference numerals denote like elements, and redundant descriptions are omitted. The present invention, however, may be embodied in various different forms, and should not be construed as being limited to only the illustrated embodiments herein. Rather, these embodiments are provided solely as examples for fully conveying the aspects and features of the present invention to those skilled in the art.

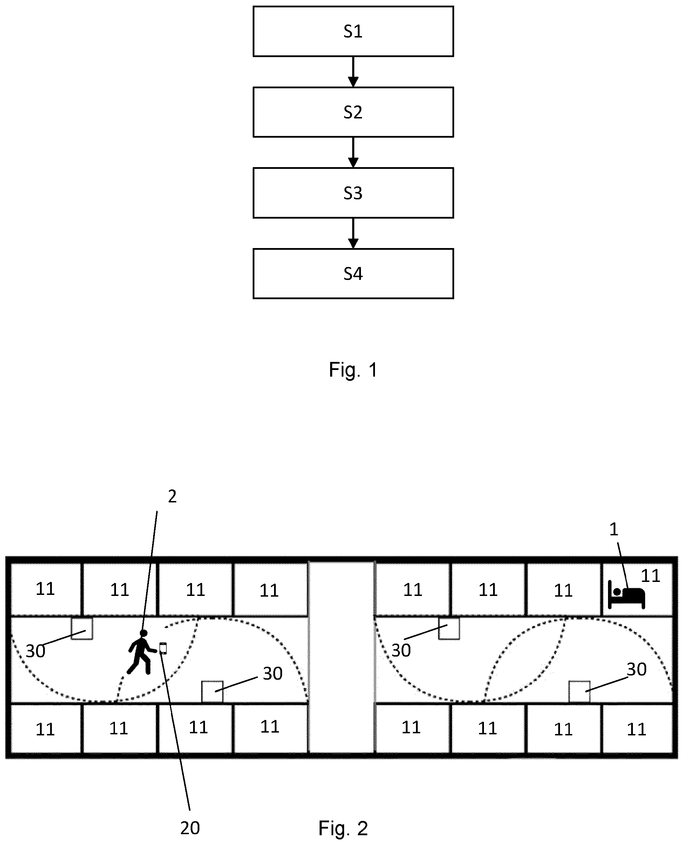

Figure 1 illustrates a method for directing a user to a subject in need of care by a controller of an electronic device according to a preferred embodiment of the disclosure. In a first step S1 information on one or more subjects in need of care is output to the user. The information in particular comprises an indication on a care need and a location of the subjects in need of care. A second step S2 of the method comprises an obtaining of information on a current location of the user. In a third step S3 a route is determined from the current location to a location of the one subject or one selected subject. In particular, the shortest route from the current location to the location of the subject is determined in the third step S3 using a shortest path algorithm. In a forth step S4 navigation information is output to the user based on the route determined in the third step S3. The navigation information in particular comprises instructions that guide the user to follow the route.

Figure 2 illustrates a system for directing a user 2 to a subject 1 in need of care according to a preferred embodiment of the disclosure. The system is illustrated in an exemplary environment, particularly one floor of a nursing home. In other words, a place of use of the system according to the disclosure is illustrated in Figure 2. The exemplary floor of the nursing home has two stations, each of which has eight rooms 11.

The system further comprises a mobile device 20, in particular an AR-enabled smartphone, hold by the user 2. The mobile device 20 comprises output means configured to display the information on the subject in need of care to the user that are output in the first step S1 of the method. The output means are further configured to display the navigation information to the user that are output in the forth step S4 of the method as shown above. The navigation information is exemplarily displayed by using augmented reality, AR.

Further the system comprises localisation means 30, that are designed here exemplarily as a plurality of beacons. In particular, two beacons are installed in the corridors of each of the stations. The beacons transmit beacon signals within a range represented by the dashed semicircles. The mobile device 20 is configured to receive the beacon signals and to determine location information of the mobile device 20 based on the beacon signals. As the mobile device 20 is held by the user 2, the location information corresponds to the current location of the user 2.

The system further comprises a controller adapted to perform the method shown in Figure 1. Parts of the controller may be formed in an edge computer (not shown) or in the mobile device 20. If the controller is not formed to any part in the mobile device 20, then the mobile device 20 functions in the system in particular as remote display and input device.

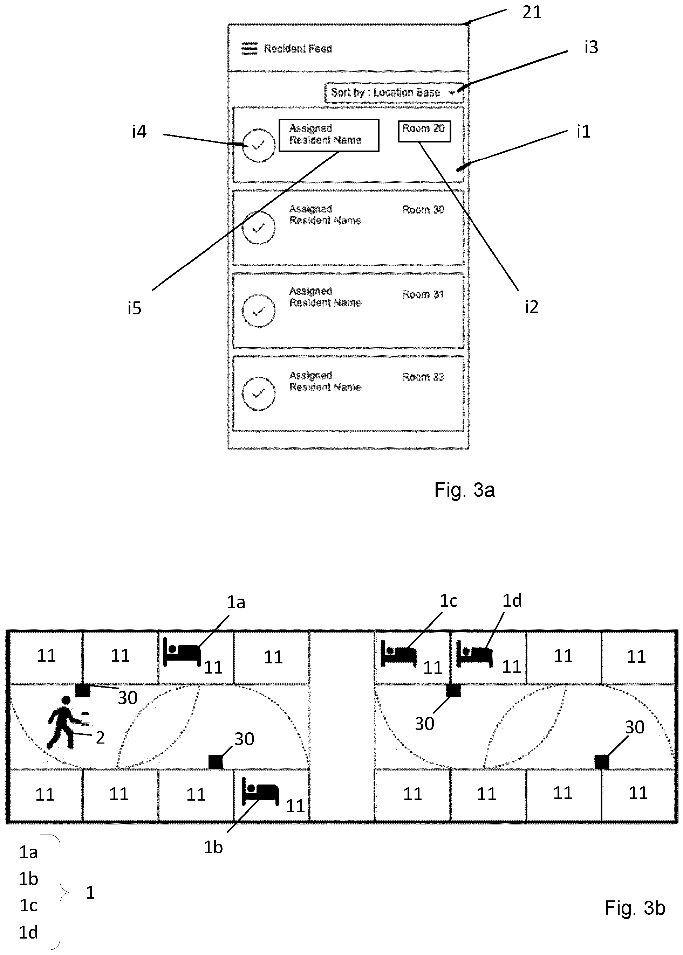

Figures 3a and 3b illustrate an outputting of information on a subject according to a method, in particular according to the first step S1 of the method in a preferred embodiment of the disclosure.

Figure 3b shows the same nursing home as in Figure 2, in which the system according to an exemplary embodiment is installed. The user 2 takes care of a plurality of subjects 1 in need of care, in particular a first subject 1a, a second subject 1b, a third subject 1c and a fourth subject 1d.

In Figure 3a, the mobile device 20 of the user 2 can be seen displaying information on the plurality of subjects in need of care using output means 21 of the mobile device 20. The information is ordered based on care needs and locations of the subjects 1a, 1b, 1c and 1d. In particular, information i1 on a subject in need of care having a shortest distance to the user and/or a highest care need is provided at the top of the order. In this case the first subject 1a was determined as the first subject in the order. The information i1 on the subject 1a comprises in particular subject location information i2 which specify the location of the subject 1a, care need information i4 which specify a care need of the subject 1a and personal information i5 which specify for example a name or an age of the subject 1a. Furthermore, sorting information i3 is displayed, which indicates criteria according to which the order of the subjects was sorted. In particular, the user may define or modify these criteria by providing a user input.

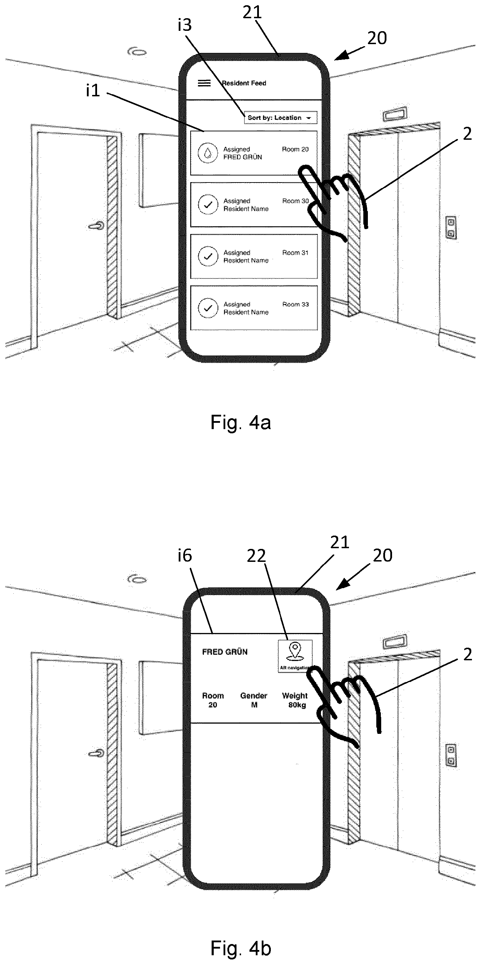

Figures 4a and 4b illustrate a user input for selecting one of a plurality of subjects 1 according to a method in a preferred embodiment of the disclosure. Figure 4a shows how the information on the plurality of subjects is displayed in a sorted manner using the output means 21 of the mobile device 20, similar to the output shown in Figure 3a. The underlying criteria for sorting the order are also output as sorting information i3. As in Figure 3a, information i1 on the first subject 1a is displayed in the first position of the order. The first subject 1a is selected based on a user input, in particular by clicking on the information i1 on the first subject 1a. The user input is represented in Figure 4a by the hand of the user 2.

In various preferred embodiments, the user input as shown in Figure 4a is sufficient to select the first subject 1a from the order and thus to start a route determination. In a further preferred embodiment, detailed information i6 on the selected subject 1a is additionally displayed, as shown in Figure 4b. By means of further user input, in particular by clicking on the virtual button 22, the user 2 can then confirm that a route to this subject 1 a is to be determined and that navigation information is to be output.

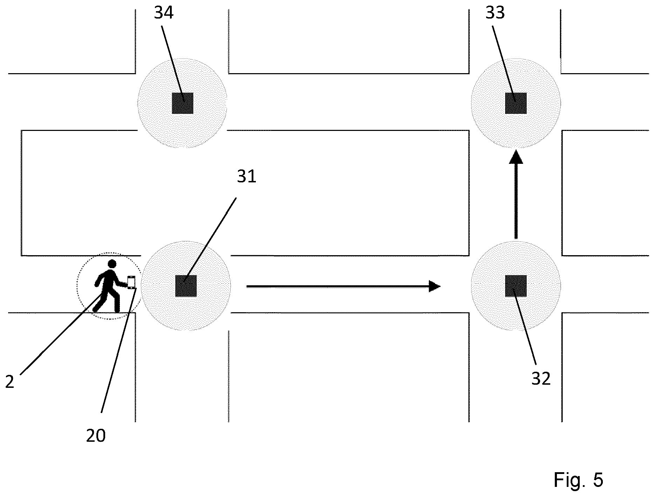

After the first subject 1a has been selected, as shown in Figures 4a and 4b, a route is determined from the current location of the user 2 to the location of the first subject 1a. In the preferred embodiment illustrated in Figure 5, the route is determined based on the Dijkstra shortest path algorithm, wherein each of a plurality of beacons is considered as one node respectively. In particular, in the environment shown in Figure 5, a first beacon 31, a second beacon 32, a third beacon 33, and a fourth beacon 34 are provided. The route is determined as the shortest path from beacon to beacon in the network of beacons 31 to 34. The arrows illustrate that here the route has been determined in particular from the first beacon 31 to the second beacon 32 and from this to the third beacon 33. The user 2 will be guided by the navigation information displayed by the mobile device 20 to follow the route represented by the arrows. In particular, whenever the user 2 gets close to a beacon, i.e. enters the circles illustrated in grey, new navigation information is displayed to guide him or her to the next beacon on the route.

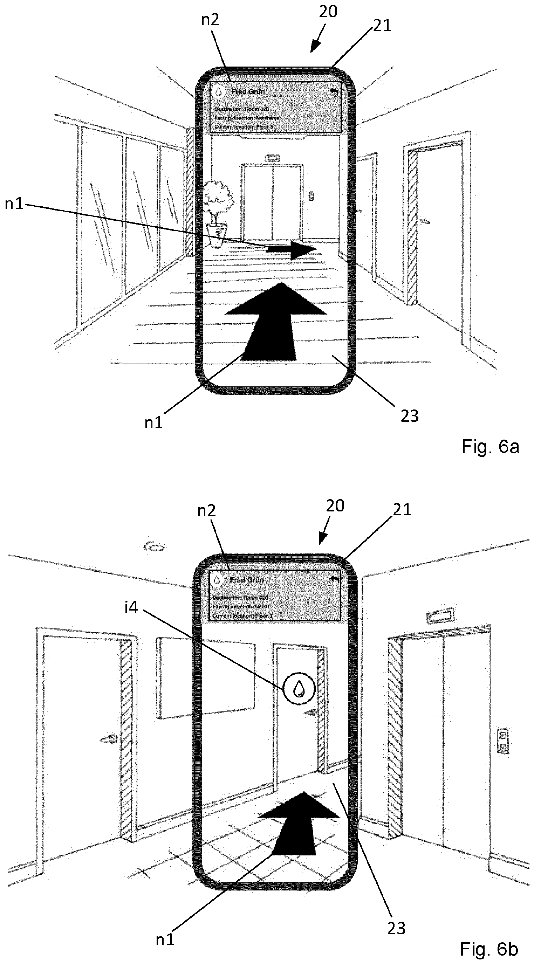

Figures 6a and 6b illustrate an outputting and in particular such a displaying of navigation information according to a method in a preferred embodiment of the disclosure. Using the output means 21, the mobile device 20 displays navigation information n1, n2, in particular first navigation information n1 and second navigation information n2. The first navigation information n1 is, in particular, AR navigation information n1 that intuitively guides the user 2 to follow the route. The first navigation information n1 is displayed overlaid with a camera image 23 of the mobile device 20 for this purpose. The second navigation information n2 further provides information about the route in text and symbol form to guide the user 2 to follow the route, as well as additional information on the subject 1a. In Figure 6b, care need information i4 are displayed as augmented reality, i.e., overlaid with the door to the room of the subject 1a, and the care need information i4 specify the care need in form of an icon, here the need to check and/or change an absorbent article of subject 1a after detecting an incontinence event.

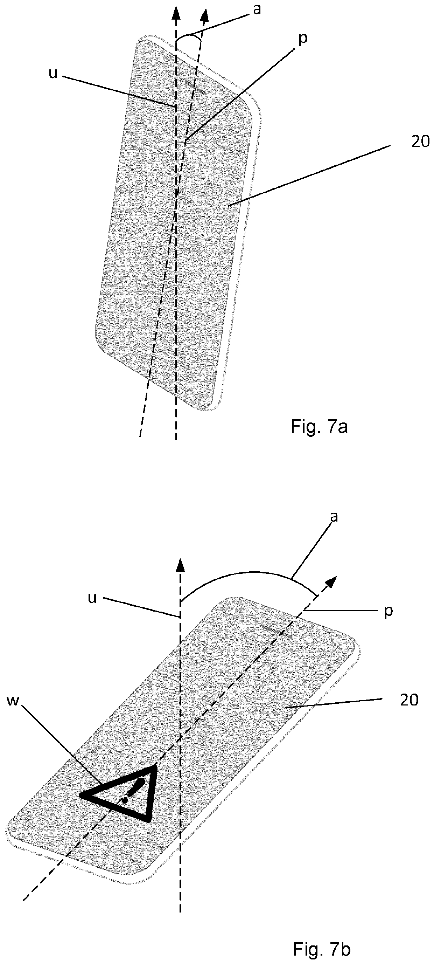

Figures 7a and 7b illustrate a determination of a pitch p by a mobile device 20 according to a preferred embodiment of the disclosure. In Figure 7a, the mobile device 20 is shown in a position in which its pitch p does not differ greatly from an upright position u. There is only a small angle a between the pitch p and the upright position u. The pitch p of the mobile device 20 shown in Figure 7b however deviates more than a predefined threshold from the upright position u. In particular, the angle a between the pitch p and the upright position u is greater than 30° here, which is why the mobile device 20 outputs a warning w instead of the navigation information. The mobile device 20 shown in Figure 7b is in a spatial position in which it cannot be ensured that an AR navigation information overlaid over a camera image obtained in this position is perceived correctly. Potential misunderstandings can thus advantageously be prevented by outputting the warning w.

### Reference list

- 1: subject
- 1a: first subject
- 1b: second subject
- 1c: third subject
- 1d: forth subject
- 2: user
- 11: room
- 20: mobile device
- 21: output means
- 22: virtual button
- 23: camera image
- 30: localisation means
- 31: first beacon
- 32: second beacon
- 33: third beacon
- 34: forth beacon
- a: angle
- i1: information on a subject
- i2: subject location information
- i3: sorting information
- i4: care need information
- i5: personal information
- i6: detailed information
- n1: first navigation information
- n2: second navigation information
- p: pitch
- S1: first step
- S2: second step
- S3: third step
- S4: forth step
- u: upright position
- w: warning

## Claims

1. Method for directing a user (2) to a subject (1) in need of care by a controller of an electronic device, the method comprising:
outputting (S1) information (i1) on the subject (1) in need of care to the user (2);
obtaining (S2) information on a current location of the user (2);
determining (S3) a route from the current location to a location of the subject (2); and
outputting (S4) navigation information (n1, n2) based on the determined route to the user (2).

2. Method according to claim 1, further comprising the steps of:
outputting information (i1) on a plurality of subjects (1) in need of care ordered based on care needs and/or locations of the subjects (1); and
receiving a user input for selecting one of the plurality of subjects (1), wherein the route is determined from the current location to the location of the selected subject.

3. Method according to claim 1 or 2, further comprising the steps of:
calculating routes from the current location to the location of each of a plurality of subjects (1); and
determining an optimized route along the locations of at least some of the plurality of subjects (1),
wherein information (i1) on the at least some of the plurality of subjects (1) in need of care is output ordered based on the optimized route and/or wherein the navigation information (n1, n2) is output based on the optimized route.

4. Method according to claim 3, wherein the optimized route is determined to be optimized with respect to its length and/or a preference for urgent care needs.

5. Method according to claim 3 or 4, wherein the optimized route comprises a supply point for receiving material and/or depositing waste.

6. Method according to any one of the preceding claims, wherein the navigation information (n1) is output using augmented reality by displaying a camera image (23) of a mobile device (20) by output means (21) thereof overlaid with the navigation information (n1).

7. System for directing a user (2) to a subject (1) in need of care, the system comprising:
a controller configured to perform a method according to any one of claims 1 to 6;
a mobile device (20) associated with the user (2) and configured to display the information (i1) on the subject in need of care and to display the navigation information (n1, n2) to the user (2), wherein the mobile device (20) comprises the controller or is configured to communicate with the controller; and
localisation means (30) configured to perform a localisation of the mobile device (20).

8. System according to claim 7, wherein the localisation means (30) comprise a plurality of beacons (31, 32, 33, 34) configured to send beacon signals to the mobile device (20), and the mobile device (20) is configured to receive the beacon signals and to determine the current location based on the beacon signals.

9. System according to claim 8, wherein the controller is configured to determine the route based on the Dijkstra shortest path algorithm, wherein each of the plurality of beacons (31, 32, 33, 34) is considered as one node respectively.

10. System according to claim 9, wherein the navigation information (n1, n2) indicates a path from the current location to a next beacon (31, 32, 33, 34) along the route.

11. System according to claim 9 or 10, wherein the controller is configured to update the current location based on determining a proximity of the mobile device (20) and one of the plurality of beacons (31, 32, 33, 34) based on the beacon signals.

12. System according to one of the claims 7 to 11 performing the method of claim 6, wherein the mobile device (20) is configured to determine a facing direction and/or a pitch (p) of the mobile device (20) and to output a warning (w) in case at least one of the following conditions is true:
the facing direction deviates more than a predetermined threshold from a direction of the navigation information (n1, n2), and
the pitch (p) indicates that the orientation of the mobile device (20) deviates more than a predetermined threshold from an upright position (u).

13. Controller for a system according to any one of claims 7 to 12, the controller being configured to perform a method according to any one of the claims 1 to 6.

14. Mobile device (20) for a system according to any one of claims 7 to 12, the mobile device (20) comprising:
a controller according to claim 13 or communication means configured for communicating with a controller according to claim 13; and
output means (21) configured to display the information (i1) on the subject in need of care and the navigation information (n1, n2) to the user (2).

15. Computer program comprising instructions which, when the program is executed by a computer, cause the computer to carry out a method according to any one of the claims 1 to 6.
